# EUROPEAN PATENT APPLICATION

(11) **EP 3 492 005 A1**
(43) Date of publication of application: **05.06.2019**
(21) Application number: 17205056.9
(22) Date of filing: 04.12.2017
(51) Int. Cl.: A61B 5/0205, A61B 5/024

(54) **A PORTABLE DEVICE AND A SYSTEM FOR MONITORING VITAL SIGNS OF A PERSON**

(71) Applicant: IMEC vzw, 3001 Leuven (BE); Katholieke Universiteit Leuven, 3000 Leuven (BE)
(72) Inventor: Torfs, Tom, 3001 Leuven (BE); Buntinx, Frank, 3001 Leuven (BE); Vaes, Bert, 3001 Leuven (BE); Van Hoof, Christiaan, 3001 Leuven (BE)
(74) Representative: AWA Sweden AB

(57) **Abstract**

A portable device for monitoring vital signs of a person, said device (102) comprising: a first wearable sensor unit (110) comprising a first electrode (114) arranged to be electrically coupled to a first limb; a second wearable sensor unit (120) comprising a second electrode (124) arranged to be electrically coupled to a second limb; wherein the first wearable sensor unit (110) and the second wearable sensor unit (120) are wiredly connected for allowing an electrocardiogram, ECG, of the person to be acquired; and wherein at least one of the first and the second wearable sensor unit (110; 120) further comprises an oximetry sensor (116; 126) arranged on the carrier (112; 122), said oximetry sensor (116; 126) including a light source (116a; 126a) for transmitting light towards the limb and a detector (116b; 126b) for detecting light being transmitted through or reflected by the limb.

## Description

### Technical field

The present inventive concept relates to a portable device and a system for monitoring vital signs of a person. In particular, the present inventive concept relates to a device that may be easily carried by a caregiver and which may be configured to be attachable to a person for monitoring the vital signs of the person.

### Background

In medical emergency rooms, bedside monitors are frequently used for monitoring primary vital signs of a patient. The primary vital signs monitored may include pulse (heart rate), blood pressure, respiratory rate, and body temperature, and may often be complemented with monitoring of electrocardiogram (ECG), heart rate variability, photoplethysmogram (PPG) and oxygen saturation. Paramedics also typically possess portable monitors that can measure some or all of these parameters in emergency situations.

However, often it is a general practitioner who is called first to a medical emergency. A general practitioner may carry a doctor's suitcase in which various equipment for assessing a patient may be stored. However, the portable monitors used by paramedics are typically far too large and heavy to carry in the doctor's suitcase. Rather, the general practitioner may therefore today typically carry a portable clip-on pulse oximeter that may be used for monitoring pulse and oxygen saturation in blood of the patient.

Nevertheless, in case of a medical emergency, having a complete monitoring functionality available provides more useful information to the practitioner which can be used to better treat the patient before the patient is transferred to an ambulance / hospital. Hence, there is a need for a monitor which is small and lightweight enough to easily fit in a doctor's suitcase and which can be easily applied to the patient without requiring complicated wiring of electrodes to the body.

### Summary

An objective of the present inventive concept is thus to provide a simple portable device, which is able to provide vital sign monitoring.

These and other objects of the present inventive concept are at least partly met by the invention as defined in the independent claims. Preferred embodiments are set out in the dependent claims.

According to a first aspect, there is provided a portable device for monitoring vital signs of a person, said device comprising: a first wearable sensor unit, the first wearable sensor unit comprising a first carrier which is configured for being arranged on a first limb of the person, the first wearable sensor unit comprising a first electrode arranged on the first carrier for being arranged to be electrically coupled to the first limb when the first carrier is arranged on the first limb; a second wearable sensor unit, the second wearable sensor unit comprising a second carrier which is configured for being arranged on a second limb of the person, the second wearable sensor unit comprising a second electrode arranged on the second carrier for being arranged to be electrically coupled to the second limb when the second carrier is arranged on the second limb; wherein the first wearable sensor unit and the second wearable sensor unit are wiredly connected to each other for sensing an electrical potential difference between the first and the second electrodes allowing an electrocardiogram, ECG, of the person to be acquired; and wherein at least one of the first and the second wearable sensor unit further comprises an oximetry sensor arranged on the carrier, said oximetry sensor including a light source for transmitting light towards the limb and a detector for detecting light being transmitted through or reflected by the limb.

The portable device of the invention thus comprises a first and a second wearable sensor unit. The first and the second wearable sensor unit may be small and lightweight, which enables the first and second wearable sensor units to be easily stored and carried in a doctor's suitcase or in a first aid kit.

The first and the second carriers may be configured for simple attachment to limbs of a person, which implies that the portable device can be quickly mounted on a person for whom monitoring of vital signs is needed.

The first wearable sensor unit and the second wearable sensor unit may be wiredly connected, e.g. by a thin, light cable. This implies that the first and second electrodes may be pre-connected and ready for acquiring an ECG without need of forming any electrical connections before starting monitoring of vital signs of the person. Thus, the portable device may be stored in a ready-to-use state.

The wired connection should at least be sufficiently long in order to allow the first wearable sensor unit and the second wearable sensor unit to be arranged on different limbs of the person. Typically, the wired connection should be at least 2 meters, in order to facilitate mounting of the first and second wearable sensor units on different limbs of the person. However, in some embodiments, the wired connection may be even shorter, such as 1.5 meters, e.g. if not all possible combinations of limb positions of the first and second wearable sensor unit need to be supported. A shorter wired connection may be advantageous in ensuring that the portable device is really small and light-weight, facilitating storing and carrying the portable device, e.g. in a doctor's suitcase or in a first aid kit.

The first and the second wearable sensor unit allows acquiring at least a single lead ECG. Thanks to one of the first and the second wearable sensor units further comprising an oximetry sensor, the portable device may acquire both a single lead ECG and information based on light interaction with blood flow, which may allow estimating oxygen saturation, but could also be used for obtaining a photoplethysmogram (PPG). Combining the ECG information with PPG information may provide extensive information of the health condition of the person so as to allow a caregiver to give the correct treatment of the person. Thus, the portable device may obtain a lot of monitoring information using two simple, small units that are easily mounted to the person.

The first and/or second electrode may be electrically coupled to a limb by means of the first and/or second electrode being in physical contact with the limb. However, the first and/or second electrode may alternatively be electrically coupled without being in direct physical contact with the limb. For instance, the first and/or second electrode may be arranged close to the limb so as to be capacitively coupled to the limb.

According to an embodiment, the first carrier is configured for being arranged on a left upper limb of the person and the second carrier is configured for being arranged on a right upper limb of the person. This implies that the first and the second electrode may be arranged so as to allow a lead-I ECG to be acquired. A lead-I ECG may allow monitoring heart functionality of a person providing quality information of a heart status of the person.

According to another embodiment, the first carrier is configured for being arranged on a left upper limb of the person and the second carrier is configured for being arranged on a left lower limb of the person. This implies that the first and the second electrode may be arranged so as to allow a lead-III ECG to be acquired.

According to yet another embodiment, the first carrier is configured for being arranged on a right upper limb of the person and the second carrier is configured for being arranged on a left lower limb of the person. This implies that the first and second electrode may be arranged so as to allow a lead-II ECG to be acquired.

It should also be realized that the first carrier and the second carrier need not necessarily be specifically configured for being arranged on a specific limb. Rather, the first carrier and the second carrier may be configured such that a user, when applying the portable device to a person may freely choose which limbs each of the carriers may be arranged on. If only one of the wearable sensor units includes an oximetry sensor, such wearable sensor units should however preferably be arranged on an upper limb. If the carriers are configured such that a user may freely choose which limb each carrier is to be arranged on, the portable device (or an external unit which receives measurement results from the portable device) may be configured to allow inputting information of which limb the carriers are arranged on, such that the signals are correctly analyzed.

The first carrier and the second carrier may be configured so as to allow arranging the first wearable sensor unit and the second wearable sensor unit in a firm relation to the limb. Thus, the carriers will securely stay in place during treatment of the person or even if the person is moving the limbs.

According to an embodiment, the first carrier and the second carrier may have interlocking elements such that the carrier may be configured to be arranged around a part of the limb with interlocking elements (e.g. hook and loop fasteners or buttons) engaged so as to lock the carrier in position around the part of the limb.

According to another embodiment, the first carrier and the second carrier may comprise a surface treated with an adhesive for attaching the carrier to skin. The surface may be protected by a removable film before use. In one embodiment, each of the first carrier and the second carrier comprises an adhesive patch for attaching the carrier to a limb of the person. When an adhesive patch is used, the oximetry sensor arranged on the carrier may be configured for detecting light reflected by the limb, so that the light source and the detector may be arranged side-by-side on the carrier.

According to another embodiment, each of the first carrier and the second carrier comprise two arms having inner and outer ends, wherein the two arms are connected at the inner ends and are biased for bringing the outer ends in contact with each other, whereby the carrier may be clipped onto a finger or a toe of the person.

This implies that the carrier may be very easily applied to the person, such that the portable device may be quickly arranged on the person so that monitoring of vital signs may be quickly initiated.

According to an embodiment, the oximetry sensor is arranged with the light source in one arm of the carrier and the detector in the other arm of the carrier for receiving light transmitted through the finger or the toe of the person. In this way, the carrier may be arranged on the person so as to place the light source and the detector in a proper relationship to each other allowing the oximetry sensor to acquire proper measurements.

It should also be realized that the first and the second carriers may not necessarily have a common configuration. Thus, for example, while one carrier may be configured to be clipped onto a finger or toe of the person, the other carrier may be in the form of an adhesive patch for attachment to skin of the person.

According to an embodiment, each of the first and the second wearable sensor unit further comprises an oximetry sensor arranged on the carrier.

This implies that two oximetry sensors may be provided in the portable device. The two oximetry sensors may provide two independent measurements that may be used for improving signal quality and reliability of measurements performed by the oximetry sensors.

According to an embodiment, each of the first and the second wearable sensor unit comprises a further electrode arranged on the carrier for being arranged to be electrically coupled to the limb when the carrier is arranged on the limb.

This implies that the portable device may be configured to acquire bio-impedance measurements. One pair of electrodes may be used for supplying a small current and pushing the small current through the body of the person. The other pair of electrodes may be used for measuring a voltage difference across the body. Thus, the electrical impedance of the body can be measured with greater accuracy than with only a single electrode per carrier.

The portable device may be configured to use the acquired bio-impedance measurements for detecting respiration functionality of the person. The electrical impedance of the person may be modulated by respiration, which implies that respiration may be monitored by acquiring bio-impedance measurements. For instance, a respiratory rate may be monitored.

It should also be realized that the portable device may be used in other ways for monitoring respiration. The respiration rate may be derived from modulations in a PPG signal acquired by the oximetry sensor. The respiration rate may also or alternatively be derived from modulations in the acquired ECG, e.g. from the lead-I ECG.

Thus, the portable device may use several methods for monitoring respiration, e.g. so as to use several different measurements to provide reliable results. Alternatively, only one method may be used.

According to an embodiment, the portable device further comprises a control unit, which is wiredly connected to the first wearable sensor unit and the second wearable sensor unit for receiving measurement signals from the first wearable sensor unit and the second wearable sensor unit. Thus, measurements may be gathered by a control unit, e.g. for storing of measurement results or processing of measurements.

The measurement signals may comprise electrical signals from the first and second electrodes. The control unit may comprise a processing unit for converting the electrical signals to an ECG.

The measurement signals may also comprise signals from the detector of the oximetry sensor, which may or may not have been pre-processed in the oximetry sensor before reaching the control unit.

The processing unit of the control unit may be configured to process received measurement signals in order to allow presenting the signals in a form that enables a caregiver to draw conclusions on the health condition of the person. The processing unit may also be configured to process the received measurement signals in order to analyze the signals, such as extracting features of the signals.

For instance, an ECG may be processed in order to identify features in the ECG, such as occurrence of Q wave, R wave and S wave. The ECG may also or alternatively be processed in order to extract higher level features, such as heart rate, heart rate variability, or respiration rate.

The signal from the oximetry sensor may be processed in order to form a PPG. The signal from the oximetry sensor may also or alternatively be processed in order to extract features from a formed PPG or directly from the detected light by the detector of the oximetry sensor. Thus, the signal from the oximetry sensor may be used for extracting heart rate, heart rate variability, oxygen saturation in blood, or respiration rate.

Thus, the control unit may extract information from the received measurement results. The extracted information may be provided as output, which may be used by a caregiver for assessing a condition of the person.

The processing unit may be implemented as a dedicated processing circuitry, as a general-purpose processing unit, which is provided with software for causing the processing unit to perform the desired signal processing, or as a combination of hardware and software.

The control unit may further be configured to provide control signals to the first wearable sensor unit and the second wearable sensor unit for controlling functionality of the sensor units. The control signals may be generated by the processing unit which may also process the received measurement signals or may be generated by a separate processing unit. The control signals may for instance initiate acquiring of measurement results by the first and the second wearable sensor units, or may request output of results from the first and the second wearable sensor units.

The control unit may also provide powering of the first and the second wearable sensor units. Thus, the control unit may comprise a battery. The control unit may be connected to the first and the second wearable sensor units for providing power from the battery to the sensor units.

According to an embodiment, the control unit is integrated in one of the first and the second wearable sensor units. Thus, the control unit may be provided within a housing of the first or the second wearable sensor unit and/or arranged on the first or the second carrier. This implies that the portable device may be formed from two sensor units connected by a cable such that all parts of the portable device may be easily and directly mounted on the person when the sensor units are arranged on the first and the second limbs.

According to another embodiment, the control unit is arranged in a separate housing and wiredly connected to each of the first and second wearable sensor units being arranged external to the housing. This may be particularly beneficial if the portable device comprises further sensor units apart from the first and the second wearable sensor units, as each further sensor unit may also be connected to the control unit.

The housing of the control unit may comprise an attachment element for enabling the housing to be firmly arranged on the person. Thus, the control unit may also be worn by the person. For instance, the attachment element may be a hook for attaching the housing to a belt or a waistband worn by the person.

The first wearable sensor unit and the second wearable sensor unit may be connected to the control unit by means of cables extending from the housing to the respective sensor unit. The housing may comprise a port or an opening for receiving the cables. The first wearable sensor unit and the second wearable sensor unit may thus be wiredly connected to each other via the control unit, by means of each of the first wearable sensor unit and the second wearable sensor unit being wiredly connected to the control unit.

According to an embodiment, the control unit comprises a communication unit for wireless communication with an external unit. Thus, the control unit may be configured to output measurement results, which may or may not have been processed by the control unit, to an external unit. The external unit may for instance be a nearby computing unit, such as a mobile phone, which may be connected to a display for allowing displaying of vital sign information of the person.

The communication unit may also be configured to communicate with an external unit for receiving control signals for controlling functionality of the portable device. Hence, the portable device may be controlled using an external unit.

According to an embodiment, the portable device may comprise a display. For instance, the display may be provided in the housing of the control unit. Thus, the portable device may be configured to display vital sign information, such that there is no need of a further external unit in order for a caregiver to be able to watch the vital sign information. However, as inclusion of a display may require a certain size of the portable device, a display may be omitted so as to ensure that the portable device small.

According to an embodiment, the control unit is configured to determine a pulse arrival time, PAT, based on the ECG acquired by the first and the second electrode and a photoplethysmogram, PPG, acquired by the oximetry sensor. Thus, measurements from the electrodes and the oximetry sensor may be used in combination in order to extract useful information of vital signs of the person.

The PAT may be determined as a time delay between an R peak of the ECG (associated with ejection of blood into the aorta), and arrival of the pulse wave in the limb, as detected by the photoplethysmogram.

According to an embodiment, the control unit is configured to receive at least one reference measurement of blood pressure for relating the at least one reference measurement to a determined PAT and wherein the control unit is further configured to determine a blood pressure trend based on subsequently determined PAT results.

The PAT measurement may be an indication of blood pressure. However, the relation between PAT results and blood pressure are highly individual. Thus, by receiving a reference measurement of blood pressure, the reference measurement may be related to a simultaneously determined PAT result. Thus, an initial calibration point may be obtained, and subsequent PAT results may be compared to the initial calibration point for determining a blood pressure trend. Hence, the portable device may with a simple calibration be configured to also provide monitoring of a blood pressure trend.

The reference measurement may be performed using a blood pressure cuff, which is normally carried in a doctor's suitcase, and hence easily at hand.

The reference measurement may be manually input to the control unit of the portable device, based on a manual reading of the blood pressure measured using the blood pressure cuff. Alternatively, the blood pressure cuff may be connected to the portable device so that the control unit may receive the reference measurement directly from the blood pressure cuff.

According to an embodiment, the portable unit further comprises a third wearable sensor unit, the third wearable sensor unit comprising a third carrier which is configured for being arranged on a third limb of the person, the third wearable sensor unit comprising a third electrode arranged on the third carrier for being arranged to be electrically coupled to the third limb when the third carrier is arranged on the third limb.

The third wearable sensor unit may be wiredly connected to a housing of the control unit. Alternatively, the third wearable sensor unit may be wiredly connected directly to one of the first and the second wearable sensor unit. Thus, the portable device may still be easy to use and simple to store in a doctor's suitcase, as all of the three wearable sensor units are physically connected and ready-to-use when stored in the doctor's suitcase.

The portable device may be configured to determine a 6-lead ECG based on receiving measurement signals from the first wearable sensor unit, the second wearable sensor unit and the third wearable sensor unit. The three electrodes may allow measuring three leads based on the electric potential difference between each pair of the three electrodes. Further, three leads may be mathematically derived so as to determine a 6-lead ECG. Hence, using a third wearable sensor unit, a high quality ECG may be acquired enabling the vital signs of the person to be followed very well.

According to a second aspect, there is provided a system for monitoring vital signs of a person, said system comprising: the portable device according to the first aspect including the third wearable sensor unit, and a chest patch sensor unit, wherein the chest patch sensor unit is configured for being attached to a chest of the person and comprises at least one patch electrode which is arranged on the chest patch sensor unit for being arranged to be electrically coupled to the chest of the person when the chest patch sensor unit is attached to the chest of the person.

The portable device may thus be part of a system having additional sensor units so as to allow acquiring of more information so that the vital signs of the person may be even better monitored. Thanks to using the portable device in combination with a chest patch sensor unit having at least one patch electrode, the ECG may be acquired with better accuracy.

The system may form a kit of parts. Thus, all parts of the system need not be physically connected, at least not before use. However, the system may be delivered in a common package, such that all parts of the system may be stored together e.g. in a doctor's suitcase so that all parts may be found together when there is a need of monitoring the vital signs of the person.

According to an embodiment, the system is configured to determine a 12-lead ECG based on measurement signals from the first wearable sensor unit, the second wearable sensor unit, the third wearable sensor unit and the chest patch sensor unit. Thus, using a chest patch sensor unit, a full 12-lead ECG may be acquired so as to provide complete knowledge of a heart ECG using a system that may be easily carried in a doctor's suitcase.

According to an embodiment, the chest patch sensor unit further comprises a temperature sensor for sensing a skin temperature when the chest patch sensor unit is attached to the chest of the person.

The sensing of the skin temperature may also allow estimating a core body temperature. Thus, in addition to monitoring vital signs relating to heart and lung functionality, the system may also determine a body temperature. This may allow the system to provide even more information pertaining to the health of the person so as to ensure that the person is properly treated or that any significant changes in vital signs may be immediately detected.

According to an embodiment, the system further comprises a forehead patch sensor unit, wherein the forehead patch sensor unit is configured for being attached to a forehead of the person and comprises at least one temperature sensor for sensing a skin temperature when the forehead patch sensor unit is attached to the forehead of the person.

The sensing of the skin temperature may also allow estimating a core body temperature. A temperature sensor arranged on the forehead may be sufficiently accurate for clinical use. The use of forehead patch may imply that temperature measurements are acquired without interfering with any measurements of heart or lung activity.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 is a schematic view of a system for monitoring of vital signs of a person.
Fig. 2 is a more detailed schematic view of components of a portable device of the system.
Fig. 3 is a schematic view of processing of measurement signals performed in the system.

### Detailed description

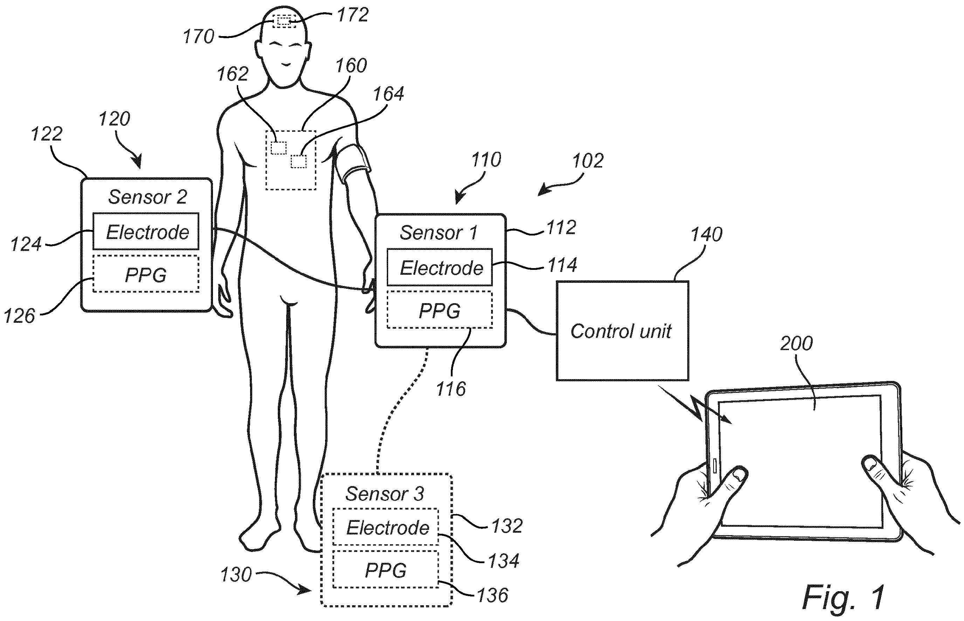

Referring now to Figs 1-2, a system 100 for monitoring of vital signs of a person will be described. The system 100 comprises a portable device 102, which is lightweight and may be take up a small space when stored and carried, such that the portable device 102 may be suitable for being carried in a doctor's suitcase.

As shown in Fig. 1, the portable device 102 may be mounted on a person in order to enable monitoring of vital signs of the person. The portable device 102 comprises a first wearable sensor unit 110 and a second wearable sensor unit 120. Optionally, the portable device 102 may further comprise a third wearable sensor unit 130.

Each wearable sensor unit 110, 120, 130 may comprise a carrier 112, 122, 132. The carrier 112, 122, 132 may be configured for being arranged on a limb of the person, so as to allow mounting the carrier 112, 122, 132 properly on the person in order to allow acquiring information for monitoring vital signs of the person.

Each wearable sensor unit 110, 120, 130 may further comprise an electrode 114, 124, 134 arranged on the carrier 112, 122, 132. The arrangement of the carrier 112, 122, 132 on a limb of the person may thus arrange the electrode 114, 124, 134 so as to be electrically coupled to the limb. The electrode 114, 124, 134 may comprise an electrically conductive material, which is exposed on a surface of the carrier 112, 122, 132 such that the electrode 114, 124, 134 may be in direct contact with skin when the carrier 112, 122, 132 is arranged on the limb. Alternatively, the electrode 114, 124, 134 may be insulated on the carrier 112, 122, 132 and may be configured to form a capacitive coupling to the limb by being sufficiently close to the limb, when the carrier 112, 122, 132 is arranged on the limb.

The electrodes 114, 124, 134 may be pairwise wiredly connected so as to allow sensing an electrical potential difference between the electrodes 114, 124, 134 such than an electrocardiogram (ECG) may be acquired.

The carriers 112, 122, 132 may be marked to provide instructions to a user as to which limb each of the carriers 112, 122, 132 is to be connected to. Thus, the carriers 112, 122, 132 may be intended to be arranged on a left arm, right arm and a left leg of the person. This implies that a lead-I ECG, a lead-II ECG and a lead-III ECG may be acquired. It should be realized that if only the first and the second wearable sensor units 110, 120 are used, these may be arranged on two different limbs in order to acquire any one of the lead-I ECG, the lead-II ECG and the lead-III ECG may be acquired.

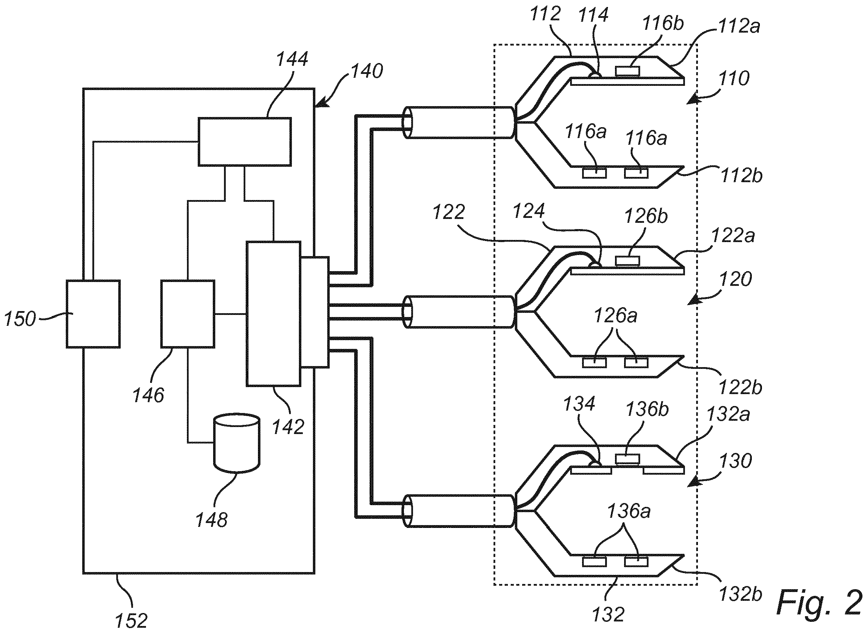

As illustrated in more detail in Fig. 2, at least one of the first, second and third wearable sensor units 110, 120, 130 may comprise an oximetry sensor 116, 126, 136. The oximetry sensor 116, 126, 136 may comprise a light source 116a, 126a, 136a and a detector 116b, 126b, 136b. The light source 116a, 126a, 136a may be configured to transmit light towards the limb and the detector 116b, 126b, 136b may be configured to detect light having interacted with the transmitted light.

The light source 116a, 126a, 136a and the detector 116b, 126b, 136b may be configured to be arranged on opposite sides of the limb when the carrier 112, 122, 132 is arranged on the limb. Thus, the detector 116b, 126b, 136b may be configured to detect light having been transmitted through the limb. Alternatively, the light source 116a, 126a, 136a and the detector 116b, 126b, 136b may be configured to be arranged on the same side of the limb when the carrier 112, 122, 132 is arranged on the limb, such that the detector 116b, 126b, 136b is configured to detect light having been reflected by the limb.

The light source 116a, 126a, 136a may be configured to emit light of two different wavelengths. For example, the light source 116a, 126a, 136a may comprise two light-emitting diodes (LED). The LEDs may e.g. be configured to emit light with a wavelength of 660 nm and 940 nm, respectively. The detector 116b, 126b, 136b may then be configured to detect an amount of light transmitted through the limb or reflected by the limb for each wavelength. A relation between the detected amounts of light for the different wavelengths may then be used in order to determine an oxygen saturation in blood of the person.

The light detected by the detector 116b, 126b, 136b may also be used for determining volumetric changes in the limb associated with blood flow, in the form of a photoplethysmogram (PPG). Thus, changes in detected amount of light may be associated with changes in blood flow and may be used for identifying heart activity. However, blood flow to the skin can also be modulated by other physiological systems, so the acquired PPG may also be used for monitoring e.g. respiration.

Oximetry sensors may be arranged in more than one wearable sensor unit, and even in each of the first, second and third wearable sensor unit 110, 120, 130. Having additional oximetry sensors 116, 126, 136 may enable acquiring several independent measurements that may be used for improving signal quality and reliability of measurements performed by the oximetry sensors 116, 126, 136.

The first, second and third wearable sensor unit 110, 120, 130 may comprise identical carriers 112, 122, 132. However, it should be realized that different types of carriers may alternatively be used.

In one embodiment, as illustrated in Fig. 2, the carrier 112, 122, 132 comprise two arms 112a, 112b; 122a, 122b; 132a, 132b having inner and outer ends, wherein the two arms are connected at the inner ends 112a, 112b; 122a, 122b; 132a, 132b and are biased for bringing the outer ends in contact with each other, whereby the carrier may be clipped onto a finger or a toe of the person. The arms 112a, 112b; 122a, 122b; 132a, 132b may for instance be spring-biased for pushing the outer ends towards contact with each other. This implies that the carrier 112, 122, 132 may provide a clip, which may be easily handled by a caregiver so as to separate the arms 112a, 112b; 122a, 122b; 132a, 132b and arrange the carrier 112, 122, 132 on a finger or toe of the person. Once applied, the caregiver may let go of the clip and the biased arrangement of the arms 112a, 112b; 122a, 122b; 132a, 132b may cause the clip to be firmly arranged on the finger or toe of the person. Hence, the carrier 112, 122, 132 may be easily applied and may ensure that the wearable sensor unit 110, 120, 130 is securely arranged on the person so that a continuous monitoring of vital signs may be provided even if the person moves.

The oximetry sensor 116, 126, 136 may be arranged with the light source 116a, 126a, 136a arranged on a surface of one arm 112a, 122a, 132a of the carrier 112, 122, 132 facing the other arm 112b, 122b, 132b, whereas the detector 116b, 126b, 136b is arranged on the other arm 112b, 122b, 132b. This implies that the detector 116b, 126b, 136b will detect light having been transmitted through the finger or toe of the person.

It should be realized that many other configurations of the carriers 112, 122, 132 may be contemplated. For instance, the carrier 112, 122, 132 may have interlocking elements such that the carrier 112, 122, 132 may be configured to be arranged around a part of the limb with interlocking elements (e.g. hook and loop fasteners or buttons) engaged so as to lock the carrier 112, 122, 132 in position around the part of the limb.

According to another embodiment, the carrier 112, 122, 132 may comprise a surface treated with an adhesive for attaching the carrier 112, 122, 132 to skin. The surface may be protected by a removable film before use so as to facilitate storing of the portable device 102.

The first, second and third wearable sensor units 110, 120, 130 may be connected by thin cables. Thus, the portable device 102 may be stored taking up a small size by folding or winding the thin cables into small-size arrangements. The size of the portable device 102 when stored, e.g. in a doctor's suitcase may thus be essentially dependent on the size of the carriers 112, 122, 132. The cables may, however, be sufficiently long in order to allow the first, second, and third wearable sensor units 110, 120, 130 to be arranged on respective limbs of the person while still being physically connected to each other.

Each of the first, second, and third wearable sensor unit 110, 120, 130 may be connected to a control unit 140. The control unit 140 may be integrated in a housing of one of the first, second and third wearable sensor units 110, 120, 130. However, the control unit 140 may alternatively be arranged in a separate physical housing, as illustrated in Fig. 1, wherein the control unit 140 is connected to the cables interconnecting the first, second and third wearable sensor units 110, 120, 130.

The control unit 140 may comprise a sensor communication unit 142, which may connect the control unit 140 to the first, second and third wearable sensor units 110, 120, 130. The sensor communication unit 142 may receive measurement signals from the first, second and third wearable sensor units 110, 120, 130 and may output instructions to the first, second and third wearable sensor units 110, 120, 130. The instructions may for instance initiate acquiring of measurement results by the first, second and third wearable sensor units 110, 120, 130, or may request output of results from the first, second wearable sensor units 110, 120, 130. The first, second and third wearable sensor units 110, 120, 130 may e.g. be connected to the sensor communication unit 142 through a port connecting the cables to the sensor communication unit 142 through a housing of the control unit 140.

The sensor communication unit 142 may thus receive measurement signals comprising electrical signals from the electrodes 114, 124, 134 which may be used by the control unit 140 in order to form ECG representations of the measurement signals.

The control unit 140 may further comprise a battery 144, which may power the control unit 140. The battery 144 may also provide power to the first, second, and third wearable sensor units 110, 120, 130 via the sensor communication unit 142 and the cables connecting the control unit 140 to the first, second and third wearable sensor units 110, 120, 130.

The control unit 140 may further comprise a processing unit 146, which may be connected to the sensor communication unit 142 and may be configured to process the measurement results received from the first, second and third wearable sensor units 110, 120, 130 via the sensor communication unit 142.

The processing unit 146 may be configured to process the received measurement results in order to convert the results into a format that may be suitable for displaying the measurement results and/or in order to extract features of the measurement results so as to provide an analysis of a health condition of the person.

The processing unit 146 may further compare extracted features to threshold levels, e.g. for identifying an abnormal situation. Thus, the processing unit 146 may be configured to output an alert signal, which may be output by the control unit 140 e.g. as a flashing light or an audio signal, so as to draw a caregiver's attention to a health condition of the person being critical.

The processing unit 146 may be any type of unit able to process information. The processing unit 146 may be a general-purpose processing unit, such as a central processing unit (CPU), which may be loaded with a computer program product in order to allow the processing unit 146 to perform the desired operations. The processing unit 146 may alternatively be a special-purpose circuitry for providing only specific logical operations. Thus, the processing unit 146 may be provided in the form of an application-specific integrated circuit (ASIC), an application-specific instruction-set processor (ASIP), a field-programmable gate array (FPGA), or a digital signal processor (DSP).

The control unit 140 may further comprise a memory 148. The memory 148 may be used for storing measurement results. Thus, measurement results acquired during monitoring of vital signs of the person may be stored in the memory 148 so as to allow later access, e.g. for reviewing a development of a condition of the person when decisions on treatment are to be taken. The memory 148 may also function as a temporary storage before the control unit 140 transmits information to an external unit 200 or as a backup memory to a memory of the external unit 200.

The control unit 140 may further comprise a communication unit 150 for communicating with the external unit 200. The communication unit 150 may be configured for wireless communication with the external unit 200 using any type of wireless communication protocol, such as Bluetooth®. The control unit 140 may be configured to transmit information through the communication unit 150 in real time, such that the external unit 200 may be used for further processing of vital signs data and/or display of the vital signs data. For instance, the communication unit 150 may be configured to communicate with a smartphone of the caregiver.

The communication unit 150 may also be configured to communicate with the external unit 200 for receiving control signals for controlling functionality of the portable device 102. Hence, the external unit 200 may provide a user interface for controlling the portable device 102.

The control unit 140 may comprise a housing 152, in which the components of the control unit 140 are arranged. The housing 152 of the control unit 140 may comprise an attachment element for enabling the housing 152 to be firmly arranged on the person. Thus, in addition to the first, second and third wearable sensor units 110, 120, 130, the control unit 140 may also be worn by the person. For instance, the attachment element may be a hook for attaching the housing 152 to a belt or a waistband worn by the person.

The control unit 140 may comprise a display integrated in the housing 152. Thus, the control unit 140 may be configured to display vital sign information, such that there is no need of a further external unit in order for a caregiver to be able to watch the vital sign information. The display may be small in order to ensure that the size of the housing 152 is small for facilitating storage and carrying of the portable device 102. Thus, the display of the control unit 140 may be configured to only display a single type of vital sign information at a time. The control unit 140 may further be provided with buttons for selecting which type of vital sign information to be displayed, such as heart rate, oxygen saturation or respiration rate.

The system 100 may optionally further comprise one or more additional sensor units 160, 170. The additional sensor units 160, 170 may or may not be physically connected to the portable device 102. For instance, the additional sensor units 160, 170 may be configured to communicate measurement results wirelessly to the control unit 140 or may be configured to communicate measurement results directly to the external unit 200.

The measurements performed by the additional sensor units 160, 170 may complement measurements performed by the portable device 102, so as to allow better information on the vital signs of the person to be acquired.

The additional sensor units 160, 170 may be stored together with the portable device, e.g. in a common package, such that all parts of the system 100 are available together and easily found, e.g. in a doctor's suitcase, when the system 100 is to be used.

The additional sensor units may comprise a chest patch sensor unit 160. The chest patch sensor unit 160 may comprise at least one patch electrode 162, which is arranged on the chest patch sensor unit 160 for being arranged to be electrically coupled to the chest of the person when the chest patch sensor unit 160 is attached to the chest of the person.

The chest patch sensor unit 160 may comprise a surface treated with an adhesive for attaching the chest patch sensor unit 160 to skin. The surface may be protected by a removable film before use so as to facilitate storing of the chest patch sensor unit 160.

The chest patch sensor unit 160 may further comprise at least one temperature sensor 164 for sensing a skin temperature when the chest patch sensor unit 160 is attached to the chest of the person.

The sensing of the skin temperature may also allow estimating a core body temperature. Thus, in addition to monitoring vital signs relating to heart and lung functionality, the system 100 may also determine a body temperature.

The additional sensor units may further comprise a forehead patch sensor unit 170. The forehead patch sensor unit 170 may comprise at least one temperature sensor 172 for sensing a skin temperature when the forehead patch sensor unit 170 is attached to the forehead of the person.

The sensing of the skin temperature may also allow estimating a core body temperature. A temperature sensor 172 arranged on the forehead may be sufficiently accurate for clinical use. The use of forehead patch may imply that temperature measurements are acquired without interfering with any measurements of heart or lung activity.

The forehead patch sensor unit 170 may comprise a surface treated with an adhesive for attaching the forehead patch sensor unit 170 to skin. The surface may be protected by a removable film before use so as to facilitate storing of the forehead patch sensor unit 170.

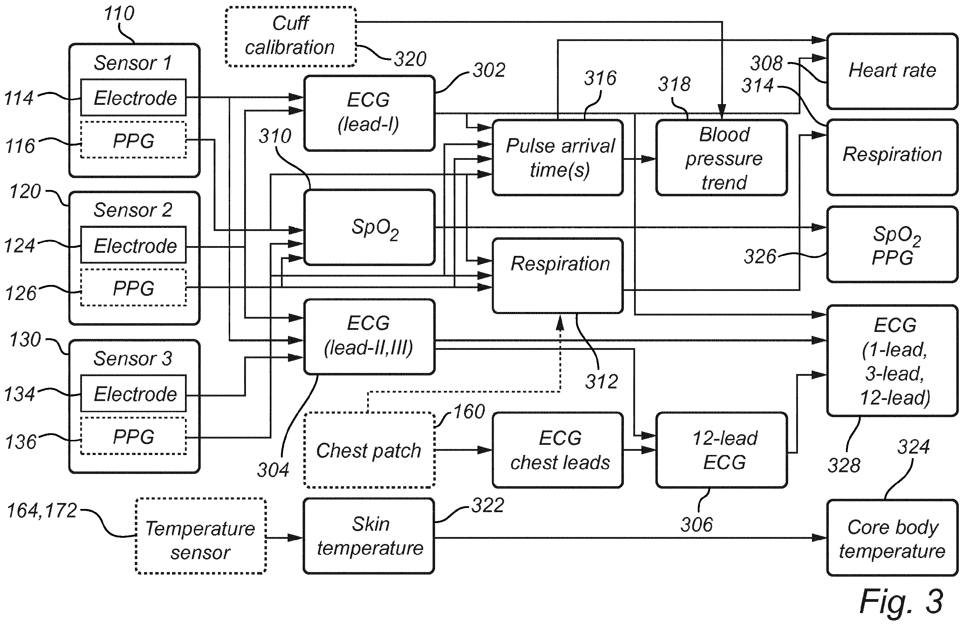

Referring now to Fig. 3, processing of measurement signals will be further described in order to discuss the vital sign information that may be extracted using the system 100.

The system 100 provides acquiring of measurement signals based on the first, second and third wearable sensor units 110, 120, 130, including electrodes 114, 124, 134, and oximetry sensors 116, 126, 136, and additional sensor units 160, 170, including electrodes 162 and temperature sensor(s) 164, 172. Sensor modalities which are optional in the system 100 are indicated by boxes with dashed lines.

The input from the electrodes 114, 124, 134 may be used for measuring lead-I ECG 302, lead-II and lead-III ECG 304 individually. Alternatively, one of the three leads may be calculated from the two other leads (lead-III = lead-II - lead-I). Based on the three determined leads, lead-I, lead-II and lead-III, three further leads, aVR, aVL and aVF, may be mathematically derived so that a 6-lead ECG may be determined.

The chest patch sensor unit 160 may comprise six precordial electrodes 162, allowing six precordial leads V1-V6 to be acquired. Thus, the measurement results from the electrodes 114, 124, 134 of the first, second and third wearable sensor units 110, 120, 130 in combination with the measurement results from the electrodes 162 of the chest patch sensor unit 160 may be used for determining a 12-lead ECG 306.

The ECG signal(s) may be used for extracting features defining the condition of the person. Thus, a heart rate 308 may be calculated based on the ECG signal(s). In addition, heart rate variability may also be determined as an indication of changes in the heart rate. The heart rate may be calculated based on a time interval between subsequent R peaks in the ECG signal.

The oximetry sensor(s) 116, 126, 136 may provide PPG signal(s). The plurality of PPG signals may be used to increase reliability of the measurements, but it may be sufficient to only use a single oximetry sensor 116 to record a single PPG signal.

The PPG signal(s) may be used for determining oxygen saturation in blood (SpO₂) 310. SpO₂ may be determined from ratios of pulsatile components in the PPG signal at different wavelengths (normally red and infrared wavelengths, but further wavelengths may also be used). An exact conversion formula from the determined ratio to the SpO₂ value may be calibrated individually for patients, but the SpO₂ determination may also be based on a pre-calibrated formula.

The PPG signal may further be processed in order to determine time intervals between beats (e.g. subsequent peaks) in the signal so as to determine a heart rate 308 and/or heart rate variability based on the PPG signal. The heart rates determined based on ECG signal(s) and PPG signal(s) may be compared in order to output a heart rate 308 with high reliability.

The PPG signal(s) may be modulated in amplitude and/or time domain due to respiratory action. Thus, respiration features 312 may also be extracted from the PPG signals. For instance, the respiration rate may be determined.

Similarly, the ECG signal(s) may also be modulated due to respiratory action. Thus, respiration features 312, such as the respiration rate, may also be extracted from the ECG signal(s). The respiration features determined based on ECG signal(s) and PPG signal(s) may be compared in order to output respiration features 314 with high reliability.

Further, the first and the second wearable sensor unit 110, 120 may each comprise a pair of electrodes. One pair of electrodes may be used for supplying a small current and pushing the small current through the body of the person. The other pair of electrodes may be used for measuring a voltage difference across the body. Thus, the bio-impedance of the body can be measured. Alternatively or additionally, the bio-impedance may be measured using a plurality of electrodes 162 in the chest patch sensor unit 160.

Bio-impedance signals may also be modulated by the respiratory action. Thus, the respiration features 312, such as the respiration rate, may also be extracted from the bio-impedance signal(s) with high accuracy and reliability. If bio-impedance signals are acquired, the respiration features determined based on bio-impedance signals may be principally used for the output respiration features 314.

The ECG signal(s) and the PPG signal(s) may further be used in combination for determining pulse arrival time(s) (PAT) 316. The PAT defines a time for blood to travel from ejection into the aorta until reaching a position in the arterial system, such as at a finger or toe where the PPG signal is acquired. The PAT 316 may be calculated as a delay between an R peak in the ECG signal(s) to a feature (e.g. foot or mid-slope of a peak) indicating pulse arrival in the PPG signal.

The PAT values may further be associated with blood pressure. However, the relation between PAT values and blood pressure may be highly individual. An initial reference measurement of blood pressure may be used for relating the PAT values of a person to blood pressure. The initial reference measurement may thus be used as an initial calibration point which further PAT values may be compared to in order to determine a blood pressure trend 318. The initial reference measurement value may be manually or automatically input to the processing unit 146 based on e.g. a blood pressure cuff measurement 320.

The blood pressure trend 318 may be useful in continuously detecting any significant changes in blood pressure. If such are detected or when otherwise appropriate, the caregiver can take another cuff measurement to confirm any such change in blood pressure. Without cuff calibration, it is expected that the PAT 316 can still be used to see significant changes in blood pressure, but not with accurate values of the blood pressure.

A skin temperature 322 may be measured by the temperature sensor 164 of the chest patch sensor unit 160 and/or the temperature sensor 172 of the forehead patch sensor unit 170. The measured skin temperature 322 may be used for estimating a core body temperature 324. Also, the temperature measurements may be used for estimating heat flux losses from the body, which may be used as input for calculating a more accurate estimate of the core body temperature 324 based on measured skin temperature 322. The heat flux losses may also be actively counteracted to remove any heat flux losses such that the measured skin temperature 322 closely approximates the core body temperature 324.

The processing of the measurement signals may thus output a plurality of types of vital sign information, such as heart rate 308, respiration features 314, SpO₂ and PPG signal 326, ECG signal(s) 328, blood pressure trend 318 and core body temperature 324.

It should be realized that further features may be extracted and output. Also, it should be realized that in some situations only some of the features are output, e.g. when not all types of sensors are available.

In the above the inventive concept has mainly been described with reference to a limited number of examples. However, as is readily appreciated by a person skilled in the art, other examples than the ones disclosed above are equally possible within the scope of the inventive concept, as defined by the appended claims.

For instance, one or more of the first, second and third wearable sensor units 110, 120, 130 may comprise a processing unit for pre-processing the acquired measurement results before measurement signals are transferred to the control unit 140. For instance, an acquired PPG signal may be pre-processed.

## Claims

1. A portable device for monitoring vital signs of a person, said device (102) comprising:
a first wearable sensor unit (110), the first wearable sensor unit (110) comprising a first carrier (112) which is configured for being arranged on a first limb of the person, the first wearable sensor unit (110) comprising a first electrode (114) arranged on the first carrier (112) for being arranged to be electrically coupled to the first limb when the first carrier (112) is arranged on the first limb;
a second wearable sensor unit (120), the second wearable sensor unit (120) comprising a second carrier (122) which is configured for being arranged on a second limb of the person, the second wearable sensor unit (120) comprising a second electrode (124) arranged on the second carrier (122) for being arranged to be electrically coupled to the second limb when the second carrier (122) is arranged on the second limb;
wherein the first wearable sensor unit (110) and the second wearable sensor unit (120) are wiredly connected to each other for sensing an electrical potential difference between the first and the second electrodes (114; 124) allowing an electrocardiogram, ECG, of the person to be acquired; and
wherein at least one of the first and the second wearable sensor unit (110; 120) further comprises an oximetry sensor (116; 126) arranged on the carrier (112; 122), said oximetry sensor (116; 126) including a light source (116a; 126a) for transmitting light towards the limb and a detector (116b; 126b) for detecting light being transmitted through or reflected by the limb.

2. The portable device according to claim 1, wherein the first carrier (112) is configured for being arranged on a left upper limb of the person and the second carrier (122) is configured for being arranged on a right upper limb of the person.

3. The portable device according to claim 1, wherein the first carrier (112) is configured for being arranged on a left upper limb of the person and the second carrier (122) is configured for being arranged on a left lower limb of the person.

4. The portable device according to any one of the preceding claims, wherein each of the first carrier (112) and the second carrier (122) comprise two arms (112a, 112b; 122a, 122b) having inner and outer ends, wherein the two arms (112a, 112b; 122a, 122b) are connected at the inner ends and are biased for bringing the outer ends in contact with each other, whereby the carrier (112; 122) may be clipped onto a finger or a toe of the person.

5. The portable device according to any one of the preceding claims, wherein each of the first and the second wearable sensor unit (110; 120) further comprises an oximetry sensor (116; 126) arranged on the carrier (112; 122).

6. The portable device according to any one of the preceding claims, wherein each of the first and the second wearable sensor unit (110; 120) comprises a further electrode arranged on the carrier (112; 122) for being arranged to be electrically coupled to the limb when the carrier (112; 122) is arranged on the limb.

7. The portable device according to any one of the preceding claims, further comprising a control unit (140), which is wiredly connected to the first wearable sensor unit (110) and the second wearable sensor unit (120) for receiving measurement signals from the first wearable sensor unit (110) and the second wearable sensor unit (120).

8. The portable device according to claim 7, wherein the control unit (140) is arranged in a separate housing (152) and wiredly connected to each of the first and second wearable sensor units (110; 120) being arranged external to the housing (152).

9. The portable device according to any one of claims 7-8, wherein the control unit (140) is configured to determine a pulse arrival time, PAT, based on the ECG acquired by the first and the second electrode and a photoplethysmogram, PPG, acquired by the oximetry sensor (116; 126).

10. The portable device according to claim 9, wherein the control unit (140) is configured to receive at least one reference measurement of blood pressure for relating the at least one reference measurement to a determined PAT and wherein the control unit (140) is further configured to determine a blood pressure trend based on subsequently determined PAT results.

11. The portable device according to any one of the preceding claims, further comprising a third wearable sensor unit (130), the third wearable sensor unit (130) comprising a third carrier (132) which is configured for being arranged on a third limb of the person, the third wearable sensor unit (130) comprising a third electrode (134) arranged on the third carrier (132) for being arranged to be electrically coupled to the third limb when the third carrier (132) is arranged on the third limb.

12. A system for monitoring vital signs of a person, said system (100) comprising:
the portable device (102) according to claim 11, and
a chest patch sensor unit (160), wherein the chest patch sensor unit (160) is configured for being attached to a chest of the person and comprises at least one patch electrode (162) which is arranged on the chest patch sensor unit (160) for being arranged to be electrically coupled to the chest of the person when the chest patch sensor unit (160) is attached to the chest of the person.

13. The system according to claim 12, wherein the system (100) is configured to determine a 12-lead ECG based on measurement signals from the first wearable sensor unit (110), the second wearable sensor unit (120), the third wearable sensor unit (130) and the chest patch sensor unit (160).

14. The system according to claim 12 or 13, wherein the chest patch sensor unit (160) further comprises a temperature sensor (164) for sensing a skin temperature when the chest patch sensor unit (160) is attached to the chest of the person.

15. The system according to any one of the preceding claims, further comprising a forehead patch sensor unit (170), wherein the forehead patch sensor unit (170) is configured for being attached to a forehead of the person and comprises at least one temperature sensor (172) for sensing a skin temperature when the forehead patch sensor unit (170) is attached to the forehead of the person.
